(19) Europäisches Patentamt
European Patent Office
Office européen des brevets

(11) **EP 4 302 701 B1**

(12) **EUROPEAN PATENT SPECIFICATION**

(45) Date of publication and mention
of the grant of the patent:
**19.03.2025 Bulletin 2025/12**

(21) Application number: **23178000.8**

(22) Date of filing: **07.06.2023**

(51) International Patent Classification (IPC):
***A61B 8/08*** (2006.01)

(52) Cooperative Patent Classification (CPC):
**A61B 8/5223; A61B 8/481; A61B 8/5276**

(54) **ULTRASOUND IMAGING SYSTEM HAVING DIGITAL ULTRASONIC IMAGING DEVICES**

ULTRASCHALLBILDGEBUNGSSYSTEM MIT DIGITALEN
ULTRASCHALLBILDGEBUNGSVORRICHTUNGEN

SYSTÈME D'IMAGERIE ULTRASONORE AYANT DES DISPOSITIFS D'IMAGERIE ULTRASONORE
NUMÉRIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **06.07.2022 PCT/CN2022/104036
09.09.2022 EP 22194740**

(43) Date of publication of application:
**10.01.2024 Bulletin 2024/02**

(73) Proprietor: **Koninklijke Philips N.V.
5656 AG Eindhoven (NL)**

(72) Inventors:
• **XU, Jingping**
**5656 AG Eindhoven (NL)**
• **LOUPAS, Thanasis**
**5656 AG Eindhoven (NL)**
• **CHEN, Zhouye**
**5656 AG Eindhoven (NL)**
• **LIN, Qizhong**
**5656 AG Eindhoven (NL)**
• **TONG, Ling**
**5656 AG Eindhoven (NL)**
• **CHEN, Jinning**
**5656 AG Eindhoven (NL)**

(74) Representative: **Philips Intellectual Property &
Standards
High Tech Campus 52
5656 AG Eindhoven (NL)**

(56) References cited:
**WO-A1-2021/224921**

• **TIYARATTANACHAI THODSAWIT ET AL: "A
Comprehensive Motion Compensation Method
for In-Plane and Out-of-Plane Motion in Dynamic
Contrast-Enhanced Ultrasound of Focal Liver
Lesions", ULTRASOUND IN MEDICINE AND
BIOLOGY., vol. 48, no. 11, 13 August 2022
(2022-08-13), US, pages 2217 - 2228,
XP093045006, ISSN: 0301-5629, DOI: 10.1016/
j.ultrasmedbio.2022.06.007**

**Description**

FIELD OF THE INVENTION

The invention relates to an ultrasound imaging system having digital ultrasonic imaging devices.

BACKGROUND OF THE INVENTION

Contrast enhanced ultrasound (CEUS) is an ultrasound imaging technique used in a variety of clinical applications. CEUS can detect the nonlinear signals received from microbubbles which circulate in the blood stream after an intravenous injection of an ultrasound contrast agent. As such CEUS imaging allows for documentation of tissue perfusion due to comparatively slow flow at the capillary level, as well as visualizing blood flow in arteries and veins. As a result, CEUS is capable at providing dynamic visualization of blood flow at both the macro- and micro-circulation levels. Among other clinical applications, CEUS imaging mode is recommended in the diagnosis and treatment of lesions on the liver, which may be malignant.

A sonographer typically operates a probe to gather images and loops that can span a few minutes. The representative images and loops gathered by the sonographer are often then sent to another location for review of the data by a radiologist or other trained clinician, for example. In some cases, a radiologist/clinician performs the sonogram, gathering/evaluating frames and loops used in diagnosis and treatment of medical condition. Moreover, radiologists/clinicians typically review cases in a remote workstation without being present during the CEUS exam which is the case at the radiology department in USA. So, the data from the procedure must be stored and transmitted from the location of the sonographer to the radiologist/clinician. This data transfer can be challenging due to the comparatively long duration of the acquired CEUS sequences.

During a CEUS procedure, scans are often taken in a two-dimensional plane through the portion of the body (e.g., the liver) being examined. A large number of frames and loops are gathered during the procedure and are sent for review by a trained clinician such as a radiologist/clinician. As will be appreciated, when a sonographer is taking a scan of a region of interest (ROI), there are many sources of movement that can impact the quality of the images being gathered. For example, movement of the patient due to breathing can result in a shift in the location of the image plane, resulting images out of the image plane of the current scan, and ultimately in images lesser quality and unproductive scans.

While some types of motion compensation are used to reduce the impact of the respiratory motion on the images being gathered, motion artifacts in the form of out-of-plane images remain when using known advanced CEUS imaging systems. Of the comparatively large amount of image data gathered in a scan, much of the data can be out-of-plane and of undesirable quality due to motion during a CEUS scan. These data are often stored in memory and are transmitted to the clinician for reviewing. As will be appreciated, more stored data or transmitted data, or both, places a burden on the computer system used to store, transmit and share the image data from the scan. These large amounts out-of-plane image data, which are of lesser quality and thus not useful to the clinician reviewing the images, are stored in ever-scarce memory. Moreover, the clinician reviewing the scans from a CEUS procedure has to sort through many images to find the images of sufficient quality to properly assess the patient's condition. As such, not only are out-of-plane image data a drain on memory resources, but also they occupy the clinician's time during review of the CEUS procedure.

The publication WO 2021/224921 A1 discloses detecting out-of-plane frames by detecting decreases in correlation values, including normalized auto correlation values. The further publication by Tiyarattanachai Thodsawit et al.: "A Comprehensive Motion Compensation Method for In-Plane and Out-of-Plane Motion in Dynamic Contrast-Enhanced Ultrasound of Focal Liver Lesions" Ultrasound in Medicine and Biology, relates to motion correction based on a local maximum normalized correlation coefficient.

What is needed is a system that overcomes at least the noted drawbacks of known systems set forth above.

SUMMARY OF THE INVENTION

The invention is defined by the independent claims. The dependent claims define advantageous embodiments.

According to an embodiment of the present disclosure, a system for providing contrast enhanced ultrasound (CEUS) images comprises: an ultrasound probe adapted to provide the ultrasound images; a processor; a tangible, non-transitory computer-readable medium that stores instructions, which when executed by the processor causes the processor to: generate time-intensity-curves for the ultrasound images; determine, based on the time-intensity-curves, out-of-plane frames of the ultrasound images; remove the out-of-plane frames from the ultrasound images based on a criterion to provide an optimized set of frames; and a display in communication with the processor and configured to display the optimized set of frames.

According to another embodiment of the present disclosure, a tangible, non-transitory computer-readable medium stores instructions, which when executed by a processor, cause the processor to: generate time-intensity-curves for the ultrasound images; determine, based on the time-intensity-curves, out-of-plane frames of contrast enhanced ultrasound images (CEUS); remove the out-of-plane frames from ultrasound images based on a criterion to provide an optimized set of frames; and a display in communication with the processor and configured to display the optimized set of frames.

According to another embodiment of the present disclosure, a method of providing ultrasound images is disclosed. In particular, the method discloses: generating time-intensity-curves for the ultrasound images; determining, based on the time-intensity-curves, out-of-plane frames of the ultrasound images; removing the out-of-plane frames from the ultrasound images based on a criterion to provide an optimized set of frames; and displaying the optimized set of frames.

These and other aspects of the invention will be apparent from and elucidated with reference to the embodiments described hereinafter.

BRIEF DESCRIPTION OF THE DRAWINGS

The representative embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.

Fig. 1 is a simplified block diagram of a CEUS imaging system for imaging a portion of a body, according to a representative embodiment.
Fig. 2A is a flow chart of a method of collecting images using the CEUS imaging system of Fig. 1 to provide frames of images for a clinician for review according to a representative embodiment.
Fig. 2B is a flow chart of a method of reviewing all cinematic loops (cine loops) collected in the method of Fig. 2A by a radiologist or other trained clinician in accordance with a representative embodiment.
Fig. 3 is a flow chart of a method of removing out-of-plane frames according to a representative embodiment.
Fig. 4 is a graph of CEUS intensity versus time (also referred to as a TIC curve) for an ideal wash-in and wash-out cycle.
Fig. 5 is a graph of CEUS intensity versus time curve (TIC curve) and a fitted TIC curve based on actual data gathered the CEUS imaging system of Fig. 1 according to a representative embodiment.
Fig. 6A is a flow chart of a method for determining whether a frame is an in-plane frame or an out-of-plane frame using changes in the TIC curve according to a representative embodiment.
Fig. 6B is a flow chart of a method of selecting representative CEUS frames or short loops at or near characteristic points of a TIC curve according to a representative embodiment.
Fig. 7 is a flow chart of a method for determining whether a frame is an in-plane frame or an out-of-plane frame using a normalized cross-correlation coefficient (NCCC) between adjacent frames according to a representative example.

DETAILED DESCRIPTION OF EMBODIMENTS

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

As described more fully below, the present teachings relate to a CEUS system, method and tangible, non-transitory computer readable medium that provide a contrast enhanced ultrasound (CEUS) workflow with representative short, limited numbers of frames, or loop selections, or both. Among other benefits, the workflow according to the present teachings reduces the time and effort needed for the review procedure since 1) a necessary subset of representative images with TIC (time intensity-curve) curves as well as two pre-contrast B-mode images are transferred to the workstation; and 2) the radiologist's or other trained clinician's effort is concentrated in reviewing comparatively smaller datasets containing essential diagnostic information, which is automatically abstracted from the entire CEUS cine-loop (cinematic loop). This novel CEUS workflow will simplify and facilitate the CEUS image acquisition and interpretation efforts. As such, the CEUS system, method and tangible, non-transitory computer readable medium that provide a CEUS workflow provide a beneficial practical application and improvements in this and potentially other technical fields.

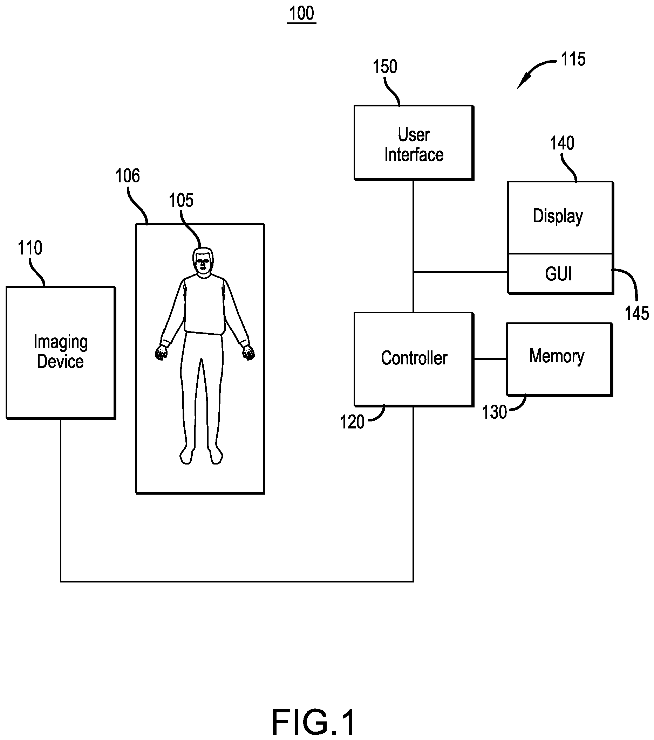

Fig. 1 is a simplified block diagram of an imaging system 100 for imaging a region of interest of a subject, according to a representative embodiment.

Referring to Fig. 1, the imaging system 100 comprises an imaging device 110 and a computer system 115 for controlling imaging of a region of interest in a patient 105 on a table 106. The imaging device 110 is illustratively an ultrasound imaging system capable of providing a contrast enhanced ultrasound (CEUS) image scan of a region of interest (ROI) of the patient 105.

The computer system 115 receives image data from the imaging device 110, and stores and processes the imaging data according to representative embodiments described herein. The computer system 115 comprises a controller or processor 120, a memory 130, a display 140 comprising a graphical user interface (GUI) 145, and a user interface 150. The display 140 may also include a loudspeaker (not shown) to provide audible feedback.

The memory 130 stores instructions executable by the controller 120. When executed, and as described more fully below, the instructions cause the controller 120 to allow the user to perform different steps using the GUI 145 or the user interface 150, or both, and, among other tasks, to initialize an ultrasound imaging device comprising a transducer. In addition, the controller 120 may implement additional operations based on executing instructions, such as instructing or otherwise communicating with another element of the computer system 115, including the memory 130 and the display 140, to perform one or more of the above-noted processes.

The memory 130 may include a main memory and/or a static memory, where such memories may communicate with each other and the controller 120 via one or more buses. The memory 130 stores instructions used to implement some or all aspects of methods and processes described herein.

As will become clearer as the present description continues, the instructions stored in memory 130 may be referred to as "modules," with different modules comprising executable instructions, which when executed by a processor, carry out the various functions described in connection with various representative embodiments described below. These modules include but are not limited to a module to automatically identify out-of-plane (OOP) frames and loops, and remove them, and a module to select representative frames and short loops of images for storing, or transmission to a radiologist or other clinician for review.

The memory 130 may be implemented by any number, type and combination of random-access memory (RAM) and read-only memory (ROM), for example, and may store various types of information, such as software algorithms, which serves as instructions, which when executed by a processor cause the processor to perform various steps and methods according to the present teachings. Furthermore, updates to the methods and processes described herein may also be provided to the computer system 115 and stored in memory 130.

The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium known in the art. The memory 130 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 130 may store software instructions and/or computer readable code that enable performance of various functions. The memory 130 may be secure and/or encrypted, or unsecure and/or unencrypted.

"Memory" is an example of computer-readable storage media, and should be interpreted as possibly being multiple memories or databases. The memory or database for instance may be multiple memories or databases local to the computer, and/or distributed amongst multiple computer systems or computing devices, or disposed in the 'cloud' according to known components and methods. Examples of computer readable storage media include non-transitory media such as computer memory devices that store information in a format that is readable by a computer or data processing system. More specific examples of non-transitory media include computer disks and non-volatile memories.

The processor or controller 120 is representative of one or more processing devices, and is configured to execute software instructions stored in memory 130 to perform functions as described in the various embodiments herein. The controller 120 may be implemented by field programmable gate arrays (FPGAs), application specific integrated circuits (ASICs), systems on a chip (SOC), a general-purpose computer, a central processing unit, a computer processor, a microprocessor, a graphics processing unit (GPU), a microcontroller, a state machine, programmable logic device, or combinations thereof, using any combination of hardware, software, firmware, hard-wired logic circuits, or combinations thereof. Additionally, any processing unit or processor herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems, such as in a cloud-based or other multi-site application. The term computing device should also be interpreted to include a collection or network of computing devices each including a processor or processors. Modules have software instructions to carry out the various functions using one or multiple processors that may be within the same computing device or which may be distributed across multiple computing devices.

The display 140 may be a monitor such as a computer monitor, a television, a liquid crystal display (LCD), a light emitting diode (LED) display, a flat panel display, a solid-state display, or a cathode ray tube (CRT) display, or an electronic whiteboard, for example. The display 140 may also provide a graphical user interface (GUI) 145 for displaying and receiving information to and from the user.

The user interface 150 may include a user and/or network interface for providing information and data output by the controller 120 and/or the memory 130 to the user and/or for receiving information and data input by the user. That is, the user interface 150 enables the user to operate the imaging device as described herein, and to schedule, control or manipulate aspects of the imaging system 100 of the present teachings. Notably, the user interface 150 enables the controller 120 to indicate the effects of the user's control or manipulation. The user interface 150 may include one or more of ports, disk drives, wireless antennas, or other types of receiver circuitry. The user interface 150 may further connect one or more interface devices, such as a mouse, a keyboard, a mouse, a trackball, a joystick, a microphone, a video camera, a touchpad, a touchscreen, voice or gesture recognition captured by a microphone or video camera, for example.

Notably, the controller 120, the memory 130, the display 140, the GUI 145 and the user interface 150 may be located away from (e.g., in another location of a building, or another building) the imaging device 110 operated by a sonographer. The controller 120, the memory 130, the display 140, the GUI 145 and the user interface 150 may be, for example, located where the radiologist/clinician is located. Notably, however, additional controllers, the memories, displays, GUI and user interfaces may be located near the sonographer and are useful in effecting the various functions of the imaging device 110 needed to complete the CEUS scans contemplated by the present teachings.

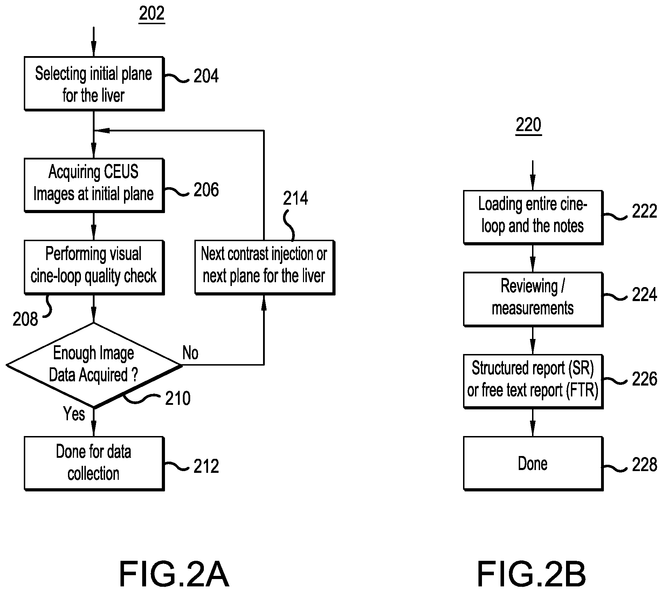

Fig. 2A is a flow chart of a method 202 of collecting images using the CEUS imaging system of Fig. 1 to provide frames of images for a clinician for review according to a representative embodiment. Various aspects and details of the method are common to those described in connection with representative embodiments of Fig. 1. These common aspects and details may not be repeated to avoid obscuring the presently described representative embodiment.

Referring to Fig. 2A, an initial plane for imaging the liver is selected at 204 and a CEUS image is acquired at the initial plane at 206. That is, at 204 the sonographer begins a CEUS scan at an initial location, such as at a lesion on the liver. In performing the scan, the imaging device 110 captures an image of a two-dimensional image plane (sometimes referred to as a slice), which is the initial plane, and acquiring a CEUS image is at the initial plane. The initial plane is located at a portion of the body selected for imaging, which for illustrative purposes may be a targeted lesion region at the middle of the ultrasound image. The CEUS image is acquired by putting the probe at a suitable position/orientation, then collecting incoming frames over the entire examination period of 3 to 6 minutes. As used herein and as described more fully below, an image taken in the desired image plane or not too far out of the desired image plane from where the sonographer is attempting to gather image data is referred to as being an in-plane (IP) image and includes the full region for the lesion to be examined, and is desirable for further review to aid in diagnosis or treatment. Notably, these desired in-plane frames may be referred to herein as optimized frames at least because they provide the radiologist/clinician with frames most useful in diagnosing and treating a patient, and do not include OOP frames, which are not only less useful in diagnosis and treatment of a patient, but also, if provided to the radiologist/clinician, may cause the radiologist/clinician to be burdened with reviewing a comparatively large number of less than optimal frames from the CEUS procedure.

However, and as described more fully below, relative movement of the patient and imaging device can cause the imaging device 110 to capture an image in another image plane that is not the same as the desired initial plane. For example, when the sonographer is attempting to capture an image at the selected location (e.g., the targeted lesion region), movement of the patient (e.g., caused by breathing) or an unintended movement of the imaging device 110 by the sonographer, the imaging device 110 will have moved relative to the selected location. This will cause the imaging device 110 to capture an ultrasound image from another plane different from initial plane. By contrast in a desired IP image, based on certain factors discussed more fully below, the image taken at another plane that is too far from the initial plane is referred to herein as being an OOP image, and is not desirable. According to various aspects of the present teachings described in connection with representative embodiments below, OOP images that are deemed too far out of the initial plane are and are not included in the images provided for review by a radiologist or similarly trained clinician. By one measure, in an OOP a significant portion (e.g., 70%-100%) of the targeted region for the lesion is lost in the current image frame.

After completion of 206, the method 202 proceeds to 208 for performing a visual cine-loop quality check. For example, the sonographer may review the images acquired in 206 to check the quality of the images gathered (e.g., in the 3-6 minute portion of the procedure as alluded to above).

At 210, the sonographer determines if the image data acquired is sufficient for a complete review and analysis of the condition of the anatomy being imaged. When the sonographer determines that enough image data have been acquired, the method 202 proceeds to 212 where the collected data are stored, or transmitted to another location for storage and review, or both.

When the sonographer determines that more image data is required, the method 202 continues at 214. Here a second contrast agent may be needed for the current plane or the next plane of the liver where the appearance of perfusion is not clear during the first injection period. The method 202 then returns to 206, and the procedure is repeated until it the sonographer determines at 210 that the image data acquired is sufficient for a complete review and analysis of the condition of the anatomy being imaged. The method 202 then proceeds to 212 where the collected data are stored, or transmitted to another location for storage and review, or both. As described more fully below, OOP images that are not useful for the desired imaging procedure are removed and not stored at 212. Rather the image data that are stored at 212 comprise only images that are beneath a threshold set for OOP images.

Fig. 2B is a flow chart of a method 220 of reviewing all cinematic loops (cine-loops) collected in the method of Fig. 2A by a radiologist or other trained clinician in accordance with a representative embodiment. Various aspects and details of the method are common to those described in connection with representative embodiments of Figs. 1 and 2A. These common aspects and details may not be repeated to avoid obscuring the presently described representative embodiment.

At 222 the entire imaging procedure, including entire cine-loops and notes from the sonographer are loaded for review by a radiologist/clinician. By way of illustration, the imaging procedure loaded at 222 may be initially stored on a suitable memory device and transported to another location wherein the sonographer is located. Alternatively, the entire imaging procedure gathered at 212 may be transmitted (e.g., by a wired or wireless communication link) and loaded at 222 for review by the radiologist/clinician. As will be appreciated, and as will become clearer as the present description continues, by the present teachings, only the IP images are stored and transmitted for loading at 222. Beneficially, compared to known systems that include OOP and IP image data for loading for review by the radiologist/clinician, only the IP images are stored or transmitted for loading at 222. This of course reduces the memory requirements of stored image data, or bandwidth requirements for transmitted image data, or both. As such, and among other benefits, the present teachings reduce the memory requirements, or the bandwidth requirements, or both, for the collection of image data to be reviewed by the radiologist/clinician.

At 224, the image data loaded at 222 are reviewed by the radiologist/clinician and measurement are taken by the radiologist/clinician from the IP images. Beneficially, because only the IP images are stored or transmitted for loading at 222, the radiologist/clinician does not have to review less than desirable images (OOP images) at 224. By contrast, image review of known CEUS imaging systems is challenging due to the CEUS loop length (often up to 5 minutes of imaging from contrast agent injection). As such, the burden of review in not just time but mental effort by the radiologist/clinician is reduced by the system and methods of the present teachings compared to known systems and methods.

At 226 a structured report (SR) or a free text report (FTR) is generated, and at 228, the method 220 of reviewing the CEUS image data is complete.

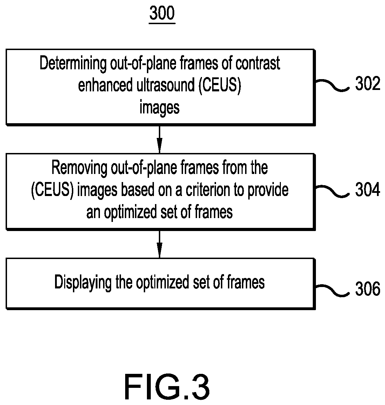

Fig. 3 is a flow chart of a method 300 of removing out-of-plane frames according to a representative embodiment. Modules comprising instructions, which when executed by the processor, cause the processor to carry out the method 300. Various aspects and details of the method are common to those described in connection with representative embodiments of Figs. 1-2B. These common aspects and details may not be repeated to avoid obscuring the presently described representative embodiment.

At 302, the method 300 begins with the determining OOP frames of a CEUS imaging procedure. As described more fully below, the determining of OOP frames is carried out according to various methods. As alluded to above, and as described more fully below, instructions comprise a module and may be stored in a tangible, non-transitory computer readable medium module that when executed by a processor cause the processor to automatically identify OOP images due to patient or imaging device motion. As noted above, and as described more fully below, the OOP images are undesired artifacts for purposes of diagnosis and treatment. At 302 these OOP images are identified for removal during data acquisition by the sonographer.

At 304 the method continues with the automatic removal of OOP frames from the CEUS imaging data based on a criterion to provide an optimized set of frames of IP images. Again, instructions stored in memory 130 comprise a module for execution by a processor to remove the OOP frames.

As noted above, this removal of OOP images is carried out while the sonographer is performing the CEUS procedure, beneficially reducing the memory requirements for storing the data of the imaging procedure or the bandwidth requirements for transmitting the data of the imaging procedure, or both. As described more fully below, the criteria upon which the decision is made to remove an image from the CEUS procedure for being an OOP procedure may be based on a comparison of normalized cross-correlation coefficients (NCCC) between adjacent frames, or from a comparison of Time Intensity Curve (TIC) data from a TIC curve and the TIC data of the frames gather during the CEUS procedure. Regardless of the type of criterion selected, a comparison to a threshold value, for example, will determine whether a particular frame should be discarded as being an OOP frame, and accordingly whether a particular frame should be saved as an IP frame for further review by the radiologist or other clinician. As such, 304 results in reduced memory requirements of the imaging system 100, or the bandwidth requirements for transmission of image data by or in the imaging system 100, or both.

At 306, the method 300 is completed by the displaying of optimized sets of frames for review by the radiologist or other clinician. By way of illustration, these optimized sets of frames may be shown on the display 140 and further manipulated by the radiologist or other clinician by the GUI 145 of the imaging system 100.

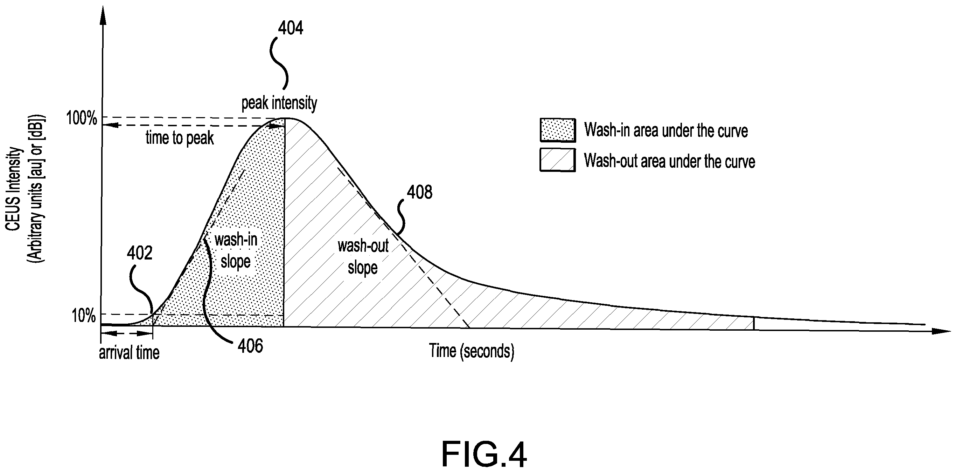

Fig. 4 is a graph of CEUS intensity versus time (also referred to as a TIC curve) for an ideal wash-in and wash-out cycle. Various aspects and details of Fig. 4 are common to those described in connection with representative embodiments of Figs. 1-3. These common aspects and details may not be repeated to avoid obscuring the presently described representative embodiment.

By the present teachings, the removal of OOP frames eliminates undesired frames and redundancies within the entire cine-loop, and leaves only IP frames for review by the sonographer or other clinician. Beneficially, the remaining IP frames/short cine-loops correspond to significant events such as a phase difference of a liver resulting in onset 402 where microbubbles in the contrast agent arrive into the targeted lesion; peak time 404 where the targeted lesion shows the strongest enhancement at CEUS image; and the middle time half 406 between the onset and peak time, which often occurs 60 seconds and 120 seconds after the onset.

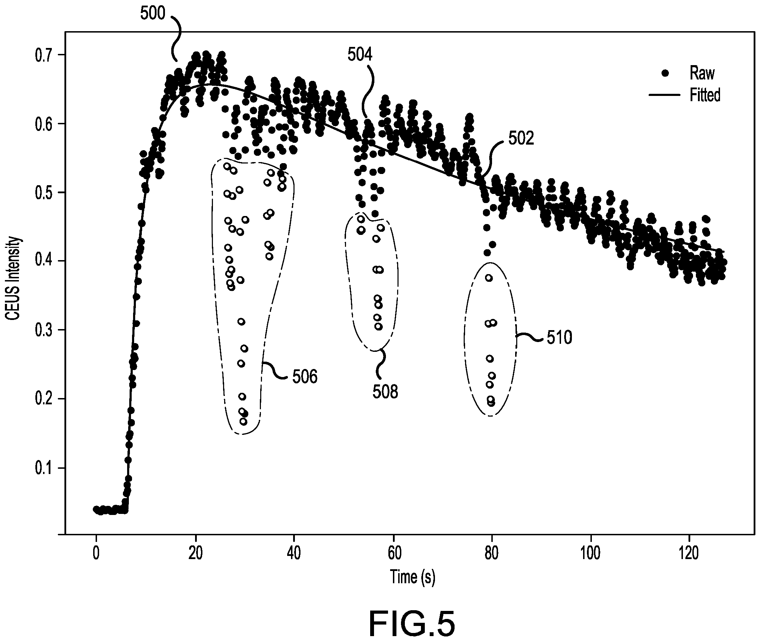

Fig. 5 is a graph of CEUS intensity versus time curve (TIC curve) and a fitted TIC curve based on data gathered the CEUS imaging system of Fig. 1 according to a representative embodiment. Various aspects and details of Fig. 5 are common to those described in connection with representative embodiments of Figs. 1-4. These common aspects and details may not be repeated to avoid obscuring the presently described representative embodiment.

Turning to Fig. 5, raw data of curve 500 are the CEUS intensity at various temporal points taken from a CEUS scan of a liver. Illustratively, these data are collected by the sonographer who has identified a targeted suspected lesion either manually or automatically. The suspected lesion can be determined from one of a number of methods such as pre-contrast B-mode image with high mechanical index (MI) (for example: MI=1.3); or from a selected frame in a CEUS loop either by its side-by-side B-mode or CEUS image, depending on the contrast ratio between lesion and background.

Notably, the raw data of curve 500 are from a relative smaller region of interest (ROI) around the targeted lesion based on the entire motion compensated CEUS loop. Fitted curve 502 is fitted curve based on the raw data of curve 500. Fitted curve 502 is made using a mathematical model specific to the anatomical part being scanned. Illustratively, the model selected to determine fitted curve 502 is a lagged normal model that determines the mean transit time (MTT) of contrast agent across the liver and is given by:

$$MTT= \mu + 1/\lambda,$$

where $\mu$ is the mean of the Lagged normal distribution; and $\lambda$ is the Péclet number, which is the ratio between the diffusive time and the convective time, estimating the contribution of both the diffusion and the convention of the microbubbles traveling through the vessels, divided by two. Further details of determining MTT values for use in connection with the present teachings may be found in "A Multi-Model Framework to Estimate Perfusion Parameters using Contrast-Enhanced Ultrasound Imaging" to Alireza Akhbardeh, et al. (Med. Phys. 46 (2), February 2019, pp. 590-600).

As will be appreciated, when applied to other anatomical elements of the body, other mathematical models, which have been found to better track the CEUS contrast intensity versus time for the specific anatomical element being studied, are used. By way of illustration and not limitation, other mathematic models include a lognormal model for the breast and heart; a gamma variate mathematic model for the carotid artery; a local density random walk (LDRW) mathematical model; and a first passage time (FTP) model for the carotid artery. These mathematic models are modules stored in memory 130 and comprise instructions, which when executed by a processor take the raw CEUS intensity data from the imaging device and calculate the fitted curve 502 for these data.

As will become clearer as the present description continues, using the systems and methods of the present teachings, average values of the data points and standard deviation from the average are determined for each data point. The average value and standard deviation are compared to a threshold to determine the data points classified as in-plane data points, and the frames of these data points will not be removed from the frame data provided to the radiologist/-clinician. By contrast, data points of that are greater that exceed the threshold are removed from the data set. By way of illustration, data points 504 that are comparatively close to the fitted curve 502 are determined by the systems and methods of the present teachings to be in-plane data points. However, data sets 506, 508, 510 likely exceed the threshold, and are likely data points from another plane erroneously captured due to relative motion of the body and imaging device 110 as discussed above. These data sets are thus determined by the system and methods of the present teachings to be OOP data points and are not stored image data of the CEUS procedure, or are not transmitted to the radiologist/clinician, or both. As noted above and as described more fully below, module to automatically identify OOP frames and loops, and remove them are stored instructions in the memory 130, which are executed by a processor to carry out this identification and removal of OOP frames and loops.

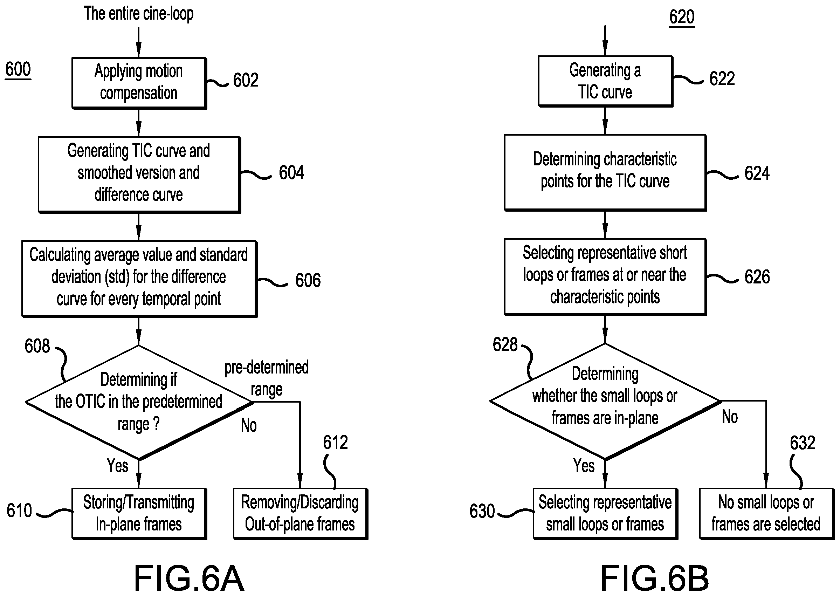

Fig. 6A is a flow chart of a method 600 for determining whether a frame is an in-plane frame or an out-of-plane frame using changes in the TIC curve according to a representative embodiment. Various aspects and details of Fig. 6A are common to those described in connection with representative embodiments of Figs. 1-5. These common aspects and details may not be repeated to avoid obscuring the presently described representative embodiment. Moreover, and as alluded to above, the method 600 is a module comprising instructions stored in memory 130. When executed by a processor, the instructions cause the processor to carry out the method 600.

At 602, a known motion compensation technique is applied to the entire cine-loop and thereby to a relatively larger region that includes the suspected lesion or whole image if necessary.

At 604 a TIC curve is generated for the comparatively smaller ROI and around the targeted lesion based on the entire motion compensated CEUS loop. By way of illustration, curve 500 is a TIC curved generated for a smaller ROI around a targeted lesion of the liver. After generation of the TIC curve, the method comprises applying a mathematical model suitable for the organ element being scanned by CEUS. Continuing with the example of Fig. 5, at this portion of the method, fitted curve 502 for the liver is generated using lagged normal model described above.

Next, at 604 of the method 600, a difference curve is generated using a difference function (diff(n)) for every temporal data point. In accordance with a representative embodiment, the fitted difference for each CEUS intensity and temporal data point n is computed as

$$\text{diff(n)= abs(OTIC(n)-FTIC(n))},$$

where OTIC is the original temporal data point, and FTIC is the fitted CEUS temporal data point n.

At 606 the method continues with the calculation of standard deviation (std) for the difference curve for every CEUS and temporal data point. In accordance with a representative embodiment, when the OTIC curve value (n) at every temporal point is outside the predetermined range the frame is deemed OOP. Just by way of illustration, the range may be expressed as (FTIC value(n)-2*std to FTIC value(n) + 2*std) as shown in Fig. 6A.

At 608 the method 600 continues with the comparison of each OTIC data point with a threshold value to see if the OTIC data point is in range. In accordance with a representative embodiment, if the value of the OTIC data point is outside a pre-determined range, the frame associated with this data point is considered to be OOP frames. Just by way of illustration, as noted above data sets 506, 508, 510 are out-of-range. As alluded to above, the predetermined range relates to OTIC data points that would be in the plane of examination of the ROI (i.e., the initial plane) where the imaging device 110 is located at the specific time in the procedure. These data points are kept (stored, or transmitted, or both). By contrast, data points that are out of range would likely be data points gathered during the specific time of the procedure, but in an image plane that differs from the initial plane due to relative motion of the imaging device 110 and the body of the patient on whom the CEUS scan is being performed.

By way of illustration, the threshold for determining whether the OITC data point is in range or out of range can be determined using (FTIC value(n)-2*std to FTIC value(n)+2*std). Data points in range are kept at 610 (stored, or transmitted to the radiologist/clinician, or both) whereas data points that exceed the predetermined range are data points of OOP frames, and are removed/discarded at 612. Notably, both fitted values and OITC values are used to determine the difference function (diff(n)), and the standard deviation is useful to identify an IP or OOP frame.

Fig. 6B is a flow chart of a method 620 of selecting representative CEUS frames or short loops at or near characteristic points of a TIC curve according to a representative embodiment. Various aspects and details of Fig. 6B are common to those described in connection with representative embodiments of Figs. 1-6A. These common aspects and details may not be repeated to avoid obscuring the presently described representative embodiment. Moreover, and as alluded to above, the method 620 is a module comprising instructions stored in memory 130. When executed by a processor, the instructions cause the processor to carry out the method 620.

In accordance with a representative embodiment, the TIC curve is determined at 622 such as described above.

At 624, the method 620 comprises determining characteristic points on the TIC curve to be analyzed. temporal points including, but not limited to, onset of the TIC curve (e.g., 402); middle of wash-in curve or maximal wash-in slope (e.g., 404); peak time of the TIC curve (e.g., 406); middle of wash-out or minimal wash-out slope (e.g., 408); a temporal point around 60 seconds based on the America College of Radiology CEUS Liver Imaging, Reporting and Data System (CEUS-LI-RADS); and a temporal point during LP or around 120 seconds if considering ACR CEUS-LI-RADs.

At 626 representative short loops or frames are selected at or near the characteristic points from 624. By selecting short cine-loops or frames from the entire cine-loop, data from important parts of the CEUS scan can be more easily isolated for review by the radiologist/clinician. Again, because the image data are reduced, less memory or less bandwidth are required for storing the data, or transmitting the data, or both.

At 628, the determination is made whether the selected short loops are IP or OOP loops. Notably, the methods for determining whether a loop comprises IP or OOP data is substantively the same as those used to determine whether a single frame comprises IP or OOP data. As will be appreciated each loop comprises a plurality of frames, so the noted methods to determine IP or OOP loops comprises repeating the method for each frame of the loop. In accordance with one representative embodiment, the determination of whether each frame of a loop is IP or OOP on an individual basis. IP frames of the loop are stored, and OOP frames of the loop are discarded. Accordingly, when the short loops are determined to be IP at 628, the method continues at 630. Otherwise, the method continues at 632.

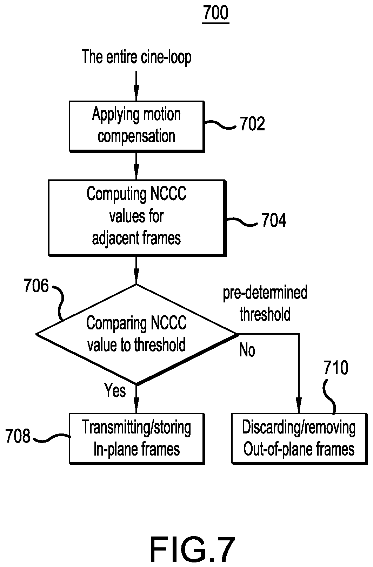

Fig. 7 is a flow chart of a method 700 for determining whether a frame is an in-plane frame or an out-of-plane frame using changes in NCCC values according to an example which is not part of the claimed invention.

Various aspects and details of Fig. 6B are common to those described in connection with representative embodiments of Figs. 1-6A. These common aspects and details may not be repeated to avoid obscuring the presently described representative example.

Moreover, and as alluded to above, the method 700 is a module comprising instructions stored in memory 130. When executed by a processor, the instructions cause the processor to carry out the method 700.

At 702, a known motion compensation method is applied to the entire cine-loop and thereby to a relatively larger region that includes the suspected lesion or whole image if necessary.

At 704 the normalized cross-correlation coefficient (NCCC) values are calculated for two adjacent frames for a targeted lesion region based on the entire motion compensated CEUS loop. Notably, adjacent frames are frames consecutive in time and frame number (e.g., frames (n-1), n, (n+1)).

In accordance with one representative example, the NCCC values ($\gamma$ (u, v)) are determined by: calculating the cross-correlation in the spatial or frequency domain, depending on the size (amount of data) of the images; calculating local sums by precomputing running sums; and using the local sums to normalize the cross-correlation to obtain the correlation coefficients. This may be expressed as:

$$\gamma(u,v) = \frac{\sum_{x,y}\left[f(x,y) - \overline{f}_{u,v}\right]\left[t(x-u, y-v) - \overline{t}\right]}{\left\{\sum_{x,y}\left[f(x,y) - \overline{f}_{u,v}\right]^2 \sum_{x,y}\left[t(x-u, y-v) - \overline{t}\right]^2\right\}^{0.5}}$$

where

- $f$ is the image.
- $\overline{t}$ is the mean of the template
- $\overline{f}_{u,v}$ is the mean of $f(x,y)$ in the region under the template.

In the equation above, f and t are functions in two spatial dimensions (x,y) and the actual values of f(x,y) and t(x,y) are used to determine the NCCC value at (x,y).

At 706 the method continues with the comparison of the NCCC values calculated in 704 to a predetermined threshold value. In accordance with a representative example, the NCCC values are calculated for any two adjacent frames for a region of interest (ROI), such as the region of the targeted lesion region based on the entire motion compensated CEUS loop. Next, out-of-plane frames are determined based on a threshold comparison and removed when they are frames of the selected frames or of the selected short loops when the NCCC value is outside the range of the threshold. Just by way of illustration, in accordance with a representative example, the determination of whether a frame is OOP is based on a pre-defined NCCC value (e.g., 0.75). When the NCCC value is less than this threshold, the frame is considered OOP and discarded. All other frames are deemed IP and are stored/shared with the clinician reviewing the scan.

If the NCCC value is large enough at 706, the data points are considered in-plane and at 708, the data points of these frames are stored, or transmitted to the radiologist/clinician, or both. If the NCCC value is less than the predetermined threshold, the frame associated with this data point is deemed to be an OOP frame and is discarded at 710.

As will be appreciated by one of ordinary skill in the art having the benefit of the present disclosure, devices, systems and methods of the present teachings provide the transmission of echo image data from an ultrasound device. For example, compared to known methods and systems, various aspects of a protocol including the beginning, duration and termination of a step in the protocol can be facilitated during the generation of the protocol, or during implementation of the protocol, or both. Moreover, errors that can result from human interaction with an imaging system can be reduced thereby reducing the need to repeat procedures, and reducing the time required to complete an imaging procedure. Notably, these benefits are illustrative, and other advancements in the field of medical imaging will become apparent to one of ordinary skill in the art having the benefit of the present disclosure.

Although methods, systems and components for implementing imaging protocols have been described with reference to several exemplary embodiments, it is understood that the words that have been used are words of description and illustration, rather than words of limitation. Changes may be made within the purview of the appended claims, as presently stated and as amended, without departing from the scope of the protocol implementation of the present teachings. The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true scope of the present disclosure. Thus, to the maximum extent allowed by law, the scope of the present disclosure is to be determined by the broadest permissible interpretation of the following claims and shall not be restricted or limited by the foregoing detailed description.

In the claims, any reference signs placed between parentheses shall not be construed as limiting the claim. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

**Claims**

1.    A system for providing ultrasound images, the system comprising:

  an ultrasound probe adapted to provide the ultrasound images;
  a processor (120) configured to:

    generate (604, 622) time-intensity-curves for the ultrasound images;
    determine (302), based on the time-intensity-curves, out-of-plane frames of the ultrasound images; and
    remove (304) the out-of-plane frames from the ultrasound images based on a criterion to provide an optimized set of frames; and

  a display (140) in communication with the processor and configured to display (306) the optimized set of frames.

2.    The system of claim 1, wherein to determine the out-of-plane frames, the processor is further configured to:
   compare time-intensity-curves between the ultrasound images.

3.    The system of claim 2, wherein to compare time-intensity-curves between the ultrasound images, the processor is further configured to:

  fit a curve of an original time-intensity-curve to determine another time-intensity-curve;
  obtain a difference function between the other time-intensity-curve and the original time-intensity-curve;
  determine (606) an average value and a standard deviation; and
  determine (608) whether the difference function at a selected frame is out of a predetermined range.

4.    The system of any of the preceding claims, wherein the display is configured to display a source set of time-sequenced ultrasound images in their entirety, and wherein the system further comprises:
   a user interface (150) configured to permit selection of the optimized set of frames.

5.    A computer program product comprising instructions, which when executed by a processor, cause the processor to:

  generate (604, 622) time-intensity-curves for the ultrasound images;
  determine (302), based the time-intensity-curves, out-of-plane frames of contrast enhanced ultrasound images;
  remove (304) the out-of-plane frames from ultrasound images based on a criterion to provide an optimized set of frames; and

display (306) the optimized set of frames.

6. The computer program product of claim 5 wherein the instructions, when executed by the processor to determine the out-of-plane frames, further cause the processor to:
compare time-intensity-curves.

7. The computer program product of claim 6, wherein the instructions, when executed by the processor to compare the time-intensity-curves between the ultrasound images, further cause the processor to:

fit a curve of an original time-intensity-curve to determine another time-intensity-curve;
obtain a difference function between the other time-intensity-curve and the original time-intensity-curve;
determine (606) an average value and a standard deviation; and
determine (608) whether the difference function at a selected frame is out of a predetermined range.

8. The computer program product of any one of claims 6 to 7, wherein the instructions, when executed by the processor to compare time-intensity-curves, further cause the processor to:

display a source set of time-sequenced ultrasound images in their entirety; and
permit selection of optimized set of frames at a user interface (150).

9. A method of providing ultrasound images, the method comprising:

generating (604, 622) time-intensity-curves for the ultrasound images;
determining (302), based on the time-intensity-curves, out-of-plane frames of the ultrasound images;
removing (304) the out-of-plane frames from the ultrasound images based on a criterion to provide an optimized set of frames; and
displaying (306) the optimized set of frames.

10. The method of claim 9. further comprising: comparing time-intensity-curves.

11. The method of claim 10. further comprising:

fitting a curve of an original time-intensity-curve to determine another time-intensity-curve ;
obtaining a difference function between the other time-intensity-curve and the original time-intensity-curve ;
determining (606) an average value and a standard deviation; and
determining (608) whether the difference function at a selected frame is out of a predetermined range.

12. The method of any one of claims 9 to 11, further comprising:

displaying a source set of time-sequenced ultrasound images in their entirety; and
permitting selection of the optimized set of frames at a user interface (150).


**Patentansprüche**

1. System zum Bereitstellen von Ultraschallbildern, wobei das System umfasst:

eine Ultraschallsonde, die dazu ausgelegt ist, die Ultraschallbilder bereitzustellen;
einen Prozessor (120), der dazu konfiguriert ist:

Zeit-Intensitäts-Kurven für die Ultraschallbilder zu erzeugen (604, 622);
auf Basis der Zeit-Intensitäts-Kurven außerhalb der Ebene liegende Einzelbilder der Ultraschallbilder zu bestimmen (302); und
auf Basis eines Kriteriums die außerhalb der Ebene liegenden Einzelbilder aus den Ultraschallbildern zu entfernen (304), um einen optimierten Satz Einzelbilder bereitzustellen; und

eine Anzeige (140), die mit dem Prozessor in Kommunikation steht und dazu konfiguriert ist, den optimierten Satz Einzelbilder anzuzeigen (306).

2. System nach Anspruch 1, wobei der Prozessor, um die außerhalb der Ebene liegenden Einzelbilder zu bestimmen, weiter dazu konfiguriert ist:
Zeit-Intensitäts-Kurven zwischen den Ultraschallbildern zu vergleichen.

3. System nach Anspruch 2, wobei der Prozessor, um Zeit-Intensitäts-Kurven zwischen den Ultraschallbildern zu vergleichen, weiter dazu konfiguriert ist:

eine Kurve einer ursprünglichen Zeit-Intensitäts-Kurve anzupassen, um eine andere Zeit-Intensitäts-Kurve zu bestimmen;
eine Differenzfunktion zwischen der anderen Zeit-Intensitäts-Kurve und der ursprünglichen Zeit-Intensitäts-Kurve zu erhalten;
einen Durchschnittswert und eine Standardabweichung zu bestimmen (606); und
zu bestimmen (608), ob die Differenzfunktion in einem ausgewählten Einzelbild außerhalb eines vorbestimmten Bereichs liegt.

4. System nach einem der vorstehenden Ansprüche, wobei die Anzeige dazu konfiguriert ist, einen Quellsatz zeitlich aufeinanderfolgender Ultraschallbilder in seiner Gesamtheit anzuzeigen, und wobei das System weiter umfasst:
eine Benutzerschnittstelle (150), die dazu konfiguriert ist, die Auswahl des optimierten Satzes Einzelbilder zu ermöglichen.

5. Computerprogrammprodukt, das Anweisungen umfasst, die, wenn sie von einem Prozessor ausgeführt werden, den Prozessor veranlassen:

Zeit-Intensitäts-Kurven für die Ultraschallbilder zu erzeugen (604, 622);
auf Basis der Zeit-Intensitäts-Kurven außerhalb der Ebene liegende Einzelbilder von kontrastverstärkten Ultraschallbildern zu bestimmen (302);
auf Basis eines Kriteriums die außerhalb der Ebene liegenden Einzelbilder aus Ultraschallbildern zu entfernen (304), um einen optimierten Satz Einzelbilder bereitzustellen; und
den optimierten Satz Einzelbilder anzuzeigen (306).

6. Computerprogrammprodukt nach Anspruch 5, wobei die Anweisungen, wenn sie vom Prozessor ausgeführt werden, um die außerhalb der Ebene liegenden Einzelbilder zu bestimmen, den Prozessor weiter veranlassen:
Zeit-Intensitäts-Kurven zu vergleichen.

7. Computerprogrammprodukt nach Anspruch 6, wobei die Anweisungen, wenn sie vom Prozessor ausgeführt werden, um die Zeit-Intensitäts-Kurven zwischen den Ultraschallbildern zu vergleichen, den Prozessor weiter veranlassen:

eine Kurve einer ursprünglichen Zeit-Intensitäts-Kurve anzupassen, um eine andere Zeit-Intensitäts-Kurve zu bestimmen;
eine Differenzfunktion zwischen der anderen Zeit-Intensitäts-Kurve und der ursprünglichen Zeit-Intensitäts-Kurve zu erhalten;
einen Durchschnittswert und eine Standardabweichung zu bestimmen (606); und
zu bestimmen (608), ob die Differenzfunktion in einem ausgewählten Einzelbild außerhalb eines vorbestimmten Bereichs liegt.

8. Computerprogrammprodukt nach einem der Ansprüche 6 bis 7, wobei die Anweisungen, wenn sie vom Prozessor ausgeführt werden, um Zeit-Intensitäts-Kurven zu vergleichen, den Prozessor weiter veranlassen:

einen Quellsatz zeitlich aufeinanderfolgender Ultraschallbilder in seiner Gesamtheit anzuzeigen; und
an einer Benutzerschnittstelle (150) die Auswahl eines optimierten Satzes Einzelbilder zu ermöglichen.

9. Verfahren zum Bereitstellen von Ultraschallbildern, wobei das Verfahren umfasst:

Erzeugen (604, 622) von Zeit-Intensitäts-Kurven für die Ultraschallbilder;
Bestimmen (302), auf Basis der Zeit-Intensitäts-Kurven, von außerhalb der Ebene liegenden Einzelbildern der Ultraschallbilder;
Entfernen (304) der außerhalb der Ebene liegenden Einzelbilder aus den Ultraschallbildern auf Basis eines Kriteriums, um einen optimierten Satz Einzelbilder bereitzustellen; und

Anzeigen (306) des optimierten Satzes Einzelbilder.

10. Verfahren nach Anspruch 9, weiter umfassend:
Vergleichen von Zeit-Intensitäts-Kurven.

11. Verfahren nach Anspruch 10, weiter umfassend:

Anpassen einer Kurve einer ursprünglichen Zeit-Intensitäts-Kurve, um eine andere Zeit-Intensitäts-Kurve zu bestimmen;
Erhalten einer Differenzfunktion zwischen der anderen Zeit-Intensitäts-Kurve und der ursprünglichen Zeit-Intensitäts-Kurve;
Bestimmen (606) eines Durchschnittswerts und einer Standardabweichung; und
Bestimmen (608), ob die Differenzfunktion in einem ausgewählten Einzelbild außerhalb eines vorbestimmten Bereichs liegt.

12. Verfahren nach einem der Ansprüche 9 bis 11, weiter umfassend:

Anzeigen eines Quellsatzes zeitlich aufeinanderfolgender Ultraschallbilder in seiner Gesamtheit; und
Ermöglichen der Auswahl des optimierten Satzes Einzelbilder an einer Benutzerschnittstelle (150).

**Revendications**

1. Système destiné à fournir des images ultrasonores, le système comprenant :

une sonde à ultrasons adaptée pour fournir les images ultrasonores ;
un processeur (120) configuré pour :

générer (604, 622) des courbes temps-intensité pour les images ultrasonores ;
déterminer (302), sur la base des courbes temps-intensité, des trames hors plan des images ultrasonores ; et
supprimer (304) les trames hors plan des images ultrasonores sur la base d'un critère pour fournir un ensemble optimisé de trames ; et

une unité d'affichage (140) en communication avec le processeur et configurée pour afficher (306) l'ensemble optimisé de trames.

2. Système selon la revendication 1, dans lequel pour déterminer les trames hors plan, le processeur est en outre configuré pour :
comparer les courbes temps-intensité entre les images ultrasonores.

3. Système selon la revendication 2, dans lequel pour comparer les courbes temps-intensité entre les images ultrasonores, le processeur est en outre configuré pour :

ajuster une courbe d'une courbe temps-intensité d'origine pour déterminer une autre courbe temps-intensité ;
obtenir une fonction de différence entre l'autre courbe temps-intensité et la courbe temps-intensité d'origine ;
déterminer (606) une valeur moyenne et un écart type ; et
déterminer (608) si la fonction de différence au niveau d'une trame sélectionnée est en dehors d'une plage prédéterminée.

4. Système selon l'une quelconque des revendications précédentes, dans lequel l'unité d'affichage est configurée pour afficher un ensemble source d'images ultrasonores séquencées dans le temps dans leur intégralité, et dans lequel le système comprend en outre :
une interface utilisateur (150) configurée pour permettre la sélection de l'ensemble optimisé de trames.

5. Produit de programme informatique comprenant des instructions qui, lorsqu'elles sont exécutées par un processeur, amènent le processeur à :

générer (604, 622) des courbes temps-intensité pour les images ultrasonores ;

déterminer (302), sur la base des courbes temps-intensité, des trames hors plan d'images ultrasonores à contraste amélioré ;

supprimer (304) les trames hors plan des images ultrasonores sur la base d'un critère pour fournir un ensemble optimisé de trames ; et

afficher (306) l'ensemble optimisé de trames.

6. Produit de programme informatique selon la revendication 5, dans lequel les instructions, lorsqu'elles sont exécutées par le processeur pour déterminer les trames hors plan, amènent en outre le processeur à :

comparer les courbes temps-intensité.

7. Produit de programme informatique selon la revendication 6, dans lequel les instructions, lorsqu'elles sont exécutées par le processeur pour comparer les courbes temps-intensité entre les images ultrasonores, amènent en outre le processeur à :

ajuster une courbe d'une courbe temps-intensité d'origine pour déterminer une autre courbe temps-intensité ;

obtenir une fonction de différence entre l'autre courbe temps-intensité et la courbe temps-intensité d'origine ;

déterminer (606) une valeur moyenne et un écart type ; et

déterminer (608) si la fonction de différence au niveau d'une trame sélectionnée est en dehors d'une plage prédéterminée.

8. Produit de programme informatique selon l'une quelconque des revendications 6 à 7, dans lequel les instructions, lorsqu'elles sont exécutées par le processeur pour comparer les courbes temps-intensité, amènent en outre le processeur à :

afficher un ensemble source d'images ultrasonores séquencées dans le temps dans leur intégralité ; et

permettre la sélection d'un ensemble optimisé de trames au niveau d'une interface utilisateur (150).

9. Procédé de fourniture d'images ultrasonores, le procédé comprenant :

la génération (604, 622) de courbes temps-intensité pour les images ultrasonores ;

la détermination (302), sur la base des courbes temps-intensité, de trames hors plan des images ultrasonores ;

la suppression (304) des trames hors plan des images ultrasonores sur la base d'un critère pour fournir un ensemble optimisé de trames ; et

l'affichage (306) de l'ensemble optimisé de trames.

10. Procédé selon la revendication 9, comprenant en outre :

la comparaison des courbes temps-intensité.

11. Procédé selon la revendication 10, comprenant en outre :

l'ajustement d'une courbe d'une courbe temps-intensité d'origine pour déterminer une autre courbe temps-intensité ;

l'obtention d'une fonction de différence entre l'autre courbe temps-intensité et la courbe temps-intensité d'origine ;

la détermination (606) d'une valeur moyenne et d'un écart type ; et

le fait de déterminer (608) si la fonction de différence au niveau d'une trame sélectionnée est ou non en dehors d'une plage prédéterminée.

12. Procédé selon l'une quelconque des revendications 9 à 11, comprenant en outre :

l'affichage d'un ensemble source d'images ultrasonores séquencées dans le temps dans leur intégralité ; et

le fait de permettre la sélection de l'ensemble optimisé de trames au niveau d'une interface utilisateur (150).

FIG.1

202

Selecting initial plane
for the liver ⟋ 204

Acquiring CEUS
Images at initial plane ⟋ 206

Performing visual
cine-loop quality check

208

Next contrast injection or
next plane for the liver

214

Enough Image
Data Acquired ?    No

210

Yes

Done for data
collection ⟋ 212

## FIG.2A

220

Loading entire cine-
loop and the notes ⟋ 222

Reviewing /
measurements ⟋ 224

Structured report (SR)
or free text report (FTR) ⟋ 226

Done ⟋ 228

## FIG.2B

<u>300</u>

```
┌─────────────────────────────────────┐
│ Determining out-of-plane frames of contrast │
│   enhanced ultrasound (CEUS)         │ ∿ 302
│             images                   │
└─────────────────────────────────────┘
                  │
                  ▼
┌─────────────────────────────────────┐
│  Removing out-of-plane frames from the │
│ (CEUS) images based on a criterion to provide │ ∿ 304
│       an optimized set of frames     │
└─────────────────────────────────────┘
                  │
                  ▼
┌─────────────────────────────────────┐
│    Displaying the optimized set of frames │ ∿ 306
└─────────────────────────────────────┘
```

# FIG.3

FIG.4

EP 4 302 701 B1

FIG.5

FIG.6B

620

Generating a
TIC curve — 622

Determining characteristic
points for the TIC curve — 624

Selecting representative short
loops or frames at or near the
characteristic points — 626

628 — Determining
whether the small loops or
frames are in-plane

No → No small loops or
frames are selected — 632

Yes → 630 — Selecting representative
small loops or frames

FIG.6A

600

The entire cine-loop

Applying motion
compensation — 602

Generating TIC curve and
smoothed version and
difference curve — 604

Calculating average value and standard
deviation (std) for the difference
curve for every temporal point — 606

608 — Determining if
the OTIC in the predetermined
range ?

pre-determined range

No → Removing/Discarding
Out-of-plane frames — 612

Yes → 610 — Storing/Transmitting
In-plane frames

<u>700</u>

The entire cine-loop

↓

Applying motion
compensation — 702

↓

Computing NCCC
values for
adjacent frames — 704

↓

706 — Comparing NCCC
value to threshold

pre-determined
threshold

No →

Yes ↓

708 — Transmitting/storing
In-plane frames

710

Discarding/removing
Out-of-plane frames

# FIG.7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021224921 A1

**Non-patent literature cited in the description**

- **TIYARATTANACHAI THODSAWIT et al.** A Comprehensive Motion Compensation Method for In-Plane and Out-of-Plane Motion in Dynamic Contrast-Enhanced Ultrasound of Focal Liver Lesions. *Ultrasound in Medicine and Biology*

- **ALIREZA AKHBARDEH et al.** A Multi-Model Framework to Estimate Perfusion Parameters using Contrast-Enhanced Ultrasound Imaging. *Med. Phys.*, February 2019, vol. 46 (2), 590-600